# EUROPEAN PATENT SPECIFICATION

(11) **EP 1 352 644 B1**
(45) Date of publication and mention of the grant of the patent: **08.10.2008**
(21) Application number: 03007353.0
(22) Date of filing: 01.04.2003
(51) Int. Cl.: A61K 8/44, A61K 8/45, A61K 8/55, A61Q 19/10, C11D 1/94, C11D 3/37, C11D 3/22

(54) **Detergent composition**
Reinigungsmittel
Composition détergente

(30) Priority: 12.04.2002 JP 2002110975; 04.10.2002 JP 2002292354
(43) Date of publication of application: 15.10.2003
(73) Proprietor: KAO CORPORATION, Tokyo 103-8210 (JP)
(72) Inventor: Inoue, Masaki, Wakayama-shi, Wakayama 640-8580 (JP); Tamura, Yoshinori, Wakayama-shi, Wakayama 640-8580 (JP); Kaneko, Youhei, Wakayama-shi, Wakayama 640-8580 (JP)
(74) Representative: HOFFMANN EITLE

(56) References cited:
- EP-A- 0 442 701
- US-A- 4 139 485
- US-A- 4 259 204
- US-A- 4 321 256
- US-A- 4 381 259
- US-A- 4 758 376
- US-A- 5 334 387

## Description

### Technical Field

The present invention relates to a detergent composition which causes less irritation to the skin or the like, is excellent in low temperature stability, foaming power and foam quality, and gives the skin or the like a good feel after use.

### Background Art

Phosphate surfactants have conventionally been employed as a main component for shampoos or skin cleansers because of a low stimulus to the skin.

Phosphate surfactants are generally available as a mixture of a monoester and a diester and alkyl phosphates inevitably contain therein a dialkyl phosphate having poor foaming power. Since foaming power and foaming quality are not sufficient when a phosphate surfactant is used singly as a main component of a detergent, a method of incorporating an auxiliary in the phosphate surfactant is proposed in order to overcome such a drawback.

The method disclosed in Japanese Patent Laid-Open No. Sho 62-138594 gazette in which a hydroxy sulfobetaine having a specific structure is used as an auxiliary is a relatively good method for reinforcing foaming power and improving low temperature stability, but is unsatisfactory in both the foaming power and foam quality.

The method disclosed in Japanese Patent Laid-Open No. Sho 62-149798 gazette in which an amide betaine compound is used as an auxiliary is relatively good from the viewpoints of foaming characteristics and improvement in a feeling after washing, but is unsatisfactory in both the foaming power and foam quality.

The method disclosed in Japanese Patent Laid-Open No. Hei 8-134496 gazette in which a hydroxy sulfobetaine having a specific amide group is used as an auxiliary is a relatively good method from the viewpoints of the foamability, foam quality and improvement in the feeling after washing, but the foaming power, foam quality and feeling after washing achieved by this method are not satisfactory.

Accordingly, an object of the present invention is to provide a detergent composition containing a phosphate surfactant, which has excellent foaming power, provides creamy foam, gives the skin or the like a good feeling after washing, has excellent low temperature stability and permits easy mixing during preparation while retaining low irritation to the skin which is characteristic to phosphate surfactants.

### Disclosure of the Invention

The present inventors have found that, if a detergent composition containing a phosphate surfactant is incorporated with a polyoxyethylene alkyl ether sulfate and one or more of sulfobetaines and alkylamide propyl betaines, the resulting composition causes less irritation to the skin, has excellent foaming power, provides creamy foam and gives the skin or the like a good feel after washing, and have completed the present invention.

Therefore, the present invention provides a detergent composition comprising the following components (A), (B) and (C):
(A) a phosphate surfactant containing, as a mixture,
   (a₁) a phosphate monoester represented by the below-described formula (1), and
   (a₂) a phosphate diester represented by the below-described formula (2): 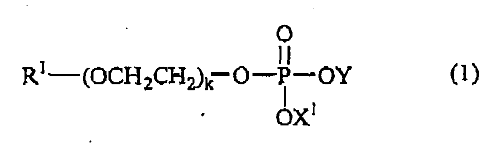 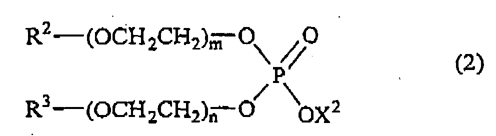 (wherein, R¹, R² and R³ each represents a linear or branched C₆-₁₈ alkyl or alkenyl group, X¹, X² and Y each represents a hydrogen atom, an alkali metal atom, an alkaline earth metal atom, an alkanolamine or ammonium, and average molar numbers k, m and n each stands for a number of from 0 to 10) at a (a₁)/(a₂) weight ratio of 85/15 to 50/50,
(B) a polyoxyethylene alkyl ether sulfate represented by the following formula (3):

   R⁴O (CH₂CH₂O)ₜSO₃M (3)

   (wherein, R⁴ represents a saturated or unsaturated C₈₋₁₈ hydrocarbon group, an average molar number t stands for a number of from 0.1 to 5 and M represents an alkali metal atom, alkanolamine or ammonium), and
(C) one or more compounds selected from sulfobetaines represented by the following formula (4): 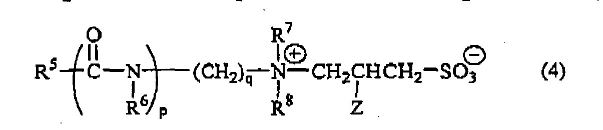 (wherein, R⁵ represents a saturated or unsaturated C₈₋₁₈ hydrocarbon group, R⁶ represents a hydrogen atom or a C₁₋₄ alkyl group, R⁷ and R⁸ each represents a C₁₋₄ alkyl group, p stands for a number of from 0 to 1, q stands for a number of from 0 to 6 and Z represents a hydrogen atom or a hydroxyl group), and alkylamide propyl betaines represented by the following formula (5): 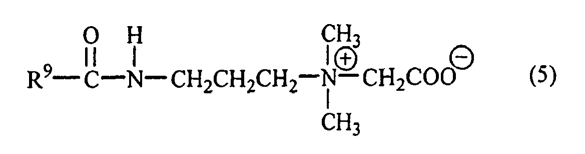 (wherein, R⁹ represents a saturated or unsaturated C₈₋₁₈ hydrocarbon group).

### Best Mode for Carrying Out the Invention

In the phosphate of the formula (1) or (2) which is a less irritative base to be used in the present invention as Component (A), a C₁₀₋₁₆, especially C₁₂₋₁₄ alkyl group is preferred as each of R¹, R² and R³, while an alkali metal atom such as potassium or sodium, or triethanolamine is preferred as each of X¹, X² and Y, with potassium being especially preferred from the viewpoint of solubility in water. The average molar numbers of ethylene oxide k, m and n are preferably 0 to 4 from the viewpoint of foaming power, with 0 to 2 being particularly preferred.

The (a₁)/(a₂) weight ratio of the alkyl phosphate monoester (a₁) and alkyl phosphate diester (a₂) contained in the Component (A) ranges from 85/15 to 50/50, preferably from 80/20 to 70/30 from the viewpoint of ease of mixing. When the content of the alkyl phosphate monoester (a₁) is lower than 50 wt.%, the foamability becomes poor. When its content exceeds 85 wt.%, on the other hand, the melting point of the alkyl phosphate mixture increases, which disturbs smooth mixing.

The content of the phosphate surfactant serving as Component (A) is preferably 5 to 40 wt.%, more preferably from 8 to 30 wt.%, particularly preferably 10 to 30 wt.% of the detergent composition in view of the foaming property, detergency and ease of mixing.

In the polyoxyethylene alkyl ether sulfate or alkyl sulfate of the formula (3) serving as Component (B), R⁴ is preferably a C₁₀₋₁₆ alkyl group, more preferably a C₁₂₋₁₄ alkyl group and particularly preferably a C₁₂ alkyl group from the viewpoint of the foaming power; the average molar number t is preferably 0.5 to 5, more preferably from 1 to 4, especially preferably from 2 to 3 from the viewpoints of low irritation and foaming power; and as M, preferred is an alkali metal, especially potassium or sodium, or triethanolamine from the viewpoints of foaming power and production cost.

The content of Component (B) in the detergent composition is preferably from 1 to 40 wt.%, more preferably from 2 to 30 wt.%, especially preferably from 3 to 10 wt.% in view of improving the foam quality of the detergent composition to creamy and in view of feeling after washing (improvement in the feel after use).

In the detergent composition of the present invention, the preferable contents of Component (A) and Component (B) are as described above. Moreover, in view of the detergency, foamability, foam quality, feeling after washing and irritation to the skin, a mixing ratio of Component (A) to Component (B) is preferably 95/50 to 50/50 by weight, with a weight ratio of 90/10 to 70/30 being particularly preferred.

In the sulfobetaine of the formula (4) serving as Component (C), it is preferred that R⁵ represents a C₈₋₁₈ alkyl group, R⁶ represents a hydrogen atom or an ethyl group, R⁷ and R⁸ each represents a methyl or ethyl group, p and q stand for 0 to 1 and 0 to 4, respectively, and Z represents a hydroxyl group.

Preferred examples of the sulfobetaine as Component (C) include alkylaminopropyl sulfobetaines, alkylhydroxy sulfobetaines, amide hydroxy sulfobetaines, and amide propyl sulfobetaines, of which lauryl dimethyl hydroxy sulfobetaine, myristyl dimethyl hydroxy sulfobetaine, N,N-dimethyl lauramide propyl-2-hydroxy sulfobetaine, and N,N-dimethyl cocamide propyl-2-hydroxy sulfobetaine are particularly preferred. Among these, lauryl dimethyl hydroxy sulfobetaine and myristyl dimethyl hydroxy sulfobetaine are most preferred.

In the alkylamide propyl betaine of the formula (5), R⁹ preferably represents a C₈₋₁₈ alkyl group. Preferred examples of the alkylamide propyl betaine as Component (C) include lauramide propyl betaine and cocamide propyl betaine.

Component (C) may be one of the above-described betaines or a mixture of at least two of these betaines. The content of Component (C) is preferably from 0.1 to 20 wt.%, more preferably from 2 to 15 wt.%, particularly preferably from 2 to 10 wt.% based on the detergent composition of the present invention from the standpoints of improvements in foamability and low temperature stability.

The weight ratio of the sum of Components (A) and (B) to Component (C), that is, [(A)+(B)]/(C) is preferably 99/1 to 40/60, especially preferably from 91/9 to 70/30 in view of improving foamability.

Combined use of Components (A), (B) and (C) can improve foamability and foam quality, which were so far insufficient in phosphate surfactants, without losing a low irritation property characteristic to the phosphate surfactants. Moreover, the combined use brings about preferable results; for example, it makes the foam creamy and after washing therewith, gives the skin or the like a moist feeling.

The detergent composition of the present invention may further contain a cationic polymer as Component (D).

Examples of the cationic polymer as Component (D) include copolymers of a quaternized vinylpyrrolidone and aminoethyl methacrylate, copolymers of adipic acid and dimethylaminohydroxypropylene diethylene triamine, poly(N,N-dimethyl-3,5-methylene piperidinium chloride) (e.g, "Merquat 100", product of NALCO CHEMICAL), copolymers of N,N-dimethyl-3,5-methylene piperidinium chloride and acrylamide (e.g., "Merquat 550", product of NALCO CHEMICAL), copolymers of acrylamide and β-methacryloxyethyltrimethylammonium, quaternized guar gum, polyethyleneimine, cationized cellulose and condensate of polyamine and polyglycol. Of these, cationic polymers having a large molecular weight (not less than 1000000) and a low cationization density (a nitrogen content of not more than 1.5%) are preferred, with cationized cellulose being particularly preferred (e.g., "POIZ-150L", product of Kao Corporation).

As Component (D), the above-described cationic polymers may be used either singly or as a mixture of at least two of them. From the viewpoints of the foamability, foam quality and feeling upon use, the content thereof is preferably from 0.05 to 0.5 wt.%, especially preferably from 0.1 to 0.3 wt.% based on the detergent composition of the present invention.

Combined use of Component (D) with Components (A), (B) and (C) brings about further improvements in foamability and foam quality.

In addition, the proper composition ratio according to the present invention suppresses a rise in viscosity during mixing, which facilitates a mixing work in the course of production.

In the detergent composition of the present invention, it is possible to incorporate, according to its using purpose, colorants, perfumes, bactericides, antiphlogistics, chelating agents, foam boosters, thickeners, viscosity modifiers, pearling agents, antiseptics, humectants, pH regulators and another surfactants within an extent not impairing the effects of the present invention.

The detergent composition of the present invention can be prepared in a conventional manner. There is no particular limitation on its form and it can be provided in any one of the conventionally known forms, for example, liquid shampoo, face wash cream, and body shampoo.

The preparation process of the detergent composition of the present invention comprises, for example, the following steps:
1) preparing a mixture containing the above-described Components (A), (B) and (C), and
2) filling a container with the resulting mixture.

Upon mixing Components (A), (B) and (C), or Components (A), (B), (C) and (D), they may be mixed in any order or at the same time. Since an increase in viscosity occurs during mixing of Component (A), stirring under heating at 40 to 80°C is preferred.

Since the detergent composition of the present invention is properly composed, a drastic increase in viscosity during mixing can be avoided and preparation can be effected while suppressing the viscosity to not more than 10000 mPa·s.

### Examples

### Examples 1 to 14 and Comparative Examples 1 to 8

Detergent compositions as shown in Tables 1 to 3 were prepared by mixing components at 70°C and adjusting the pH to 7 to 8. The state during preparation, foaming power, foamability, foam quality, feeling after washing and low temperature stability were evaluated as described below. Results are shown in Tables 1 to 3.

### (Evaluation Method)

### (1) State during preparation

The state of each detergent composition during preparation was visually evaluated in accordance with the following criteria:
A: The composition was mixed readily.
B: The composition was not mixed owing to an increase in viscosity.

### (2) Test on foaming power

As an artificial dirt, 0.5% of lanolin was added to a 1% aqueous solution (4° hard water) of each detergent composition. The resulting mixture was stirred in a cylinder for 5 minutes at 40°C by a flat propeller at a rotational speed of 1000 rpm. The rotation of the propeller was reversed at every 10 seconds. The foaming power was evaluated by the quantity of foam when the stirring was completed.

### (3) Foamability, foam quality and feeling after washing

A panel of 10 experts was asked to evaluate, in accordance with the below-described evaluation criteria, the quantity of foam, foam quality and feeling after washing when 3 mL of each detergent composition prepared in a conventional manner was applied to their palms and their hands and arms were washed therewith.

### (Foamability)

A: The quantity of foam is very large.
B: The quantity of foam is large.
C: The quantity of foam is slightly small.
D: The quantity of foam is small.

### (Foam quality)

A: Good foam quality with fine and very creamy foam.
B: Good foam quality with creamy foam.
C: Foam quality with slightly creamy foam.
C: Foam quality with light and coarse foam.

### (Feeling after washing)

A: The skin is sufficiently moist to the touch.
B: The skin is moist to the touch.
C: The skin is slightly moist to the touch.
D: The skin is not moist to the touch.

### (4) Test on low temperature stability

Each detergent composition was placed in a glass bottle. After preservation at 5°C for 10 days, the composition was taken out from the bottle and allowed to stand at room temperature (25°C) for 1 hour. The appearance of the detergent composition was visually evaluated in accordance with the following criteria:
A: The composition is transparent.
B: The composition becomes slightly turbid.
C: The composition becomes turbid to some extent.
D: Separation occurs owing to precipitation.

**Table 1**

| Component (%) | | Examples | | | | | | |
|---|---|---|---|---|---|---|---|---|
| | | 1 | 2 | 3 | 4 | 5 | 6 | 7 |
| (A) | Potassium lauryl phosphate / Potassium dilauryl phosphate (monolauryl phosphate / dilauryl phosphate = 75/25) | 16 | 16 | 16 | 16 | - | - | 16 |
| | Potassium lauryl phosphate / Potassium dilauryl phosphate (monolauryl phosphate / dilauryl phosphate = 80/20) | - | - | - | - | 14 | - | - |
| | Triethanolamine lauryl phosphate / triethanolamine dilauryl phosphate (monolauryl phosphate dilauryl phosphate = 70/30) | - | - | - | - | - | 28 | - |
| | Potassium polyoxyethylene (2) lauryl phosphate Potassium polyoxyethylene (2) dilauryl phosphate (monolauryl phosphate /dilauryl phosphate = 75/25) | - | - | - | - | - | - | - |
| | Potassium polyoxyethylene (2) myristyl phosphate / Potassium polyoxyethylene (2) dimyristyl phosphate (monomyristyl phosphate / dimyristyl phosphate = 75/25) | - | - | - | - | - | - | - |
| | Potassium polyoxyethylene (1) lauryl phosphate / Potassium polyoxyethylene (1) dilauryl phosphate (monolauryl phosphate / dilauryl phosphate = 75/25) | - | - | - | - | - | - | - |
| | Potassium polyoxyethylene (3) lauryl phosphate / Potassium polyoxyethylene (3) dilauryl phosphate (monolauryl phosphate / dilauryl phosphate = 80/20) | - | - | - | - | - | - | - |
| | Triethanolamine lauryl phosphate / triethanolamine dilauryl phosphate (monolauryl phosphate / dilauryl phosphate =100/0) | - | - | - | - | - | - | - |
| | Potassium lauryl phosphate / Potassium dilauryl phosphate (monolauryl phosphate dilauryl phosphate = 35/65) | - | - | - | - | - | - | - |
| (B) | Sodium polyoxyethylene lauryl ether sulfate (t =2 moles) | 4 | - | 4 | 4 | 5 | - | 7 |
| | Sodium polyoxyethylene lauryl ether sulfate (t = 3 moles) | - | 4 | - | - | - | - | - |
| | Trielhanolamine polyoxyethylene lauryl ether sulfate (t= 3 moles) | - | - | - | - | - | 4 | - |
| (C) | Lauryl hydroxy sulfobetaine | 5 | 5 | - | - | - | - | - |
| | Myristyl hydroxy sulfobetaine | - | - | 5 | - | - | 7 | - |
| | Lauramide propyl betaine | - | - | - | 4 | - | - | - |
| | Cocamide propyl betaine | - | - | - | - | 4 | - | - |
| | N,N-dimethyl lauramide propyl-2-hydroxy sulfobelaine | - | - | - | - | - | - | 5 |
| Purified water | | Balance | Balance | Balance | Balance | Balance | Balance | Balance |
| State during preparation | | A | A | A | A | A | A | A |
| Foaming power (mL) | | 215 | 210 | 201 | 215 | 215 | 220 | 220 |
| Foamability | | A | A | A | A | A | A | A |
| Foam quality | | A | A | A | A | A | A | B |
| Feeling after washing | | A | A | A | B | B | A | B |
| Low temperature stability | | B | A | B | B | B | A | B |

**Table 2:**

| Component (%) | | Examples | | | | | |
|---|---|---|---|---|---|---|---|
| | | 8 | 9 | 10 | 11 | 12 | 13 |
| (A) | Potassium lauryl phosphate/ Potassium dilauryl phosphate (monolauryl phosphate / dilauryl phosphate = 75125) | 14 | - | - | - | - | - |
| | Potassium lauryl phosphate / Potassium dilauryl phosphate (monolauryl phosphate / dilauryl phosphate = 80/20) | - | - | - | - | - | - |
| | Triethanolamine lauryl phosphate / triethanolamine dilauryl phosphate (monolauryl phosphate I dilauryl phosphate = 70/30) | - | - | - | - | - | - |
| | Potassium polyoxyethylene (2) Lauryl phosphate I Potassium polyoxyethylene (2) dilauryl phosphate (monolauryl phosphate 1 (dilauryl phosphate = 75/25) | - | 16 | 12 | - | - | 16 |
| | Potassium Polyoxyethylene (2) myristyl phosphate / Potassium polyoxyethylene (2) dimyristyl phosphate (monomyristyl phosphate / dimyristyl Phosphate =75/25) | - | - | 4 | - | - | - |
| | Potassium polyoxyethylene (1) lauryl phosphate I Potassium polyoxyethylene (1) dilauryl phosphate (monolauryl phosphates / dilauryl phosphate = 75/25) | - | - | - | 16 | - | - |
| | Potassium polyoxyethylene (3) lauryl phosphate / Potassium polyoxyethylene (3) dilauryl phosphate (monolauryl phosphate / dilauryl phosphate = 80/20) | - | - | - | - | 16 | - |
| | Trielhanolamine lauryl phosphate / triethanolamine dilauryl phosphate (monolauryl phosphate / dilauryl phosphate = 100/0) | - | - | - | - | - | - |
| | Potassium lauryl phosphate / Potassium dilauryl phosphate monolauryl phosphate dilauryl phosphate = 35:65) | - | - | - | - | - | - |
| (B) | Sodium polyoxyethylene lauryl ether sulfate (t = 2 moles) | - | 4 | 4 | 4 | 4 | 5 , |
| | Sodium polyoxyethylene lauryl ether sulfate (t = 3 moles) | - | - | - | - | - | - |
| | Triethanolamine polyoxyethylene lauryl ether sulfate (t =3 moles) | 4 | - | - | - | - | - |
| (C) | Lauryl hydroxy sulfobetaine | 2.5 | 5 | 5 | 5 | 5 | - |
| | Myristyl hydroxy sulfobetaine | - | - | - | - | - | - |
| | Lauramide propyl betaine | 2.5 | - | - | - | - | - |
| | Cocamide propyl betaine | - | - | - | - | - | - |
| | N,N-dimethyl lauramide propyl-2-hydroxy sulfobetaine | - | - | - | - | - | 5 |
| Purified water | | Balance | Balance | Balance | Balance | Balance | Balance |
| State during preparation | | A | A | A | A | A | A |
| Foaming power (mL) | | 205 | 215 | 220 | 215 | 204 | 211 |
| Foamability | | A | A | A | A | A | A |
| Foam quality | | A | A | A | A | A | A |
| Feeling after washing | | A | A | A | A | A | A |
| Low temperature stability | | B | A | A | B | A | B |

**Table 3**

| Component (%) | | Comparative Examples | | | | | | | |
|---|---|---|---|---|---|---|---|---|---|
| | | 1 | 2 | 3 | 4 | 5 | 6 | 7 | 8 |
| (A) | Potassium lauryl phosphate / Potassium dilauryl phosphate (monolauryl phosphate / dilauryl phosphate = 75/25) | 16 | 16 | 16 | - | 16 | - | - | - |
| | Potassium lauryl phosphate / Potassium dilauryl phosphate (monolauryl phosphate / dilauryl phosphate = 80/20) | - | - | - | - | - | - | - | - |
| | Triethanolamine lauryl phosphate / triethanolamine dilauryl phosphate (monolauryl phosphate / dilauryl phosphate = 70/30) | - | - | - | - | - | - | - | |
| | Potassium polyoxyethylene (2) lauryl phosphate / Potassium polyoxyethylene (2) dilauryl phosphate (monolauryl phosphate / dilauryl phosphate = 75/25) | - | - | - | - | - | - | - | 16 |
| | Potassium polyoxyethylene (2) myristyl phosphate I Potassium polyoxyethylene (2) dimyristyl phosphate (monomyristyl phosphate /dimyristyl phosphate = 75/25) | - | - | - | - | - | - | - | - |
| | Potassium polyoxyethylene (1) lauryl phosphate / Potassium polyoxyethylene (1) dilauryl phosphate (monolauryl phosphate / dilauryl phosphate = 75/25) | - | - | - | - | - | - | 16 | - |
| | Potassium polyoxyethylene (3) lauryl phosphate / Potassium polyoxyethylene (3) dilauryl phosphate (monolauryl phosphate / dilauryl phosphate = 80/20) | - | - | - | - | - | - | - | - |
| | Triethanolamine lauryl phosphate / triethanolamine dilauryl phosphate (monolauryl phosphate / dilauryl phosphate =100/0) | - | - | - | - | - | 28 | - | - |
| | Potassium lauryl phosphate / Potassium dilauryl phosphate (monolauryl phosphate / dilauryl phosphate = 35/65) | - | - | - | 16 | - | - | - | - |
| (B) | Sodium polyoxyethylene lauryl ether sulfate (t= 2 moles) | - | - | 4 | - | - | - | - | - |
| | Sodium polyoxyethylene lauryl ether sulfate (t= 3 moles) | - | - | - | 4 | - | - | - | - |
| | Triethanolamine polyoxyethylene lauryl ether sulfate (t = 3 moles) | - | - | - | - | - | 4 | - | - |
| (C) | Lauryl hydroxy sulfobetaine | 5 | - | - | 5 | - | - | 5 | - |
| | Myristyl hydroxy sulfobetaine | | - | - | - | - | 7 | - | - |
| | Lauramide propyl betaine | | 4 | - | - | - | - | - | 5 |
| | Cocamide propyl betaine | | - | - | - | - | - | - | - |
| | N,N-dimethyl lauramide propyl-2-hydroxy sulfobetaine | | - | - | - | 5 | - | | - |
| Purified water | | Balance | Balance | Balance | Balance | Balance | Balance | Balance | Balance |
| State during preparation | | A | A | A | A | A | B | A | A |
| Foaming power (mL) | | 150 | 165 | 110 | 85 | 161 | - | 140 | 135 |
| Foamability | | B | B | C | D | B | - | B | B |
| Foam quality | | D | D | B | D | C | - | D | D |
| Feeling after washing | | D | D | B | A | C | - | D | D |
| Low temperature stability | | B | B | B | C | B | - | B | B |

### Example 15 (Body shampoo)

The body shampoo was prepared in accordance with the following formulation.

| | (parts by weight) |
|---|---|
| Potassium lauryl phosphate/potassium dilauryl phosphate (monolauryl phosphate / dilauryl phosphate = 75/25) | 15 |
| Sodium polyoxyethylene lauryl ether sulfate (t = 2 moles) | 5 |
| Propylene glycol | 5 |
| Cationized cellulose ("Poiz C-150L", product of Kao Corporation) | 0.3 |
| Lauryl hydroxy sulfobetaine | 5 |
| Ethylene glycol distearate | 2 |
| Methylparaben | 0.2 |
| Propylparaben | 0.1 |
| BHT | 0.2 |
| Perfume | 0.05 |
| Purified water | Balance |
| Total | 100.0 |

### Example 16 (Body shampoo)

The body shampoo was prepared in accordance with the following formulation.

| | (parts by weight) |
|---|---|
| Potassium lauryl phosphate/potassium dilauryl phosphate (monolauryl phosphate / dilauryl phosphate = 75/25) | 18 |
| Sodium polyoxyethylene lauryl ether sulfate (t = 3 moles) | 5 |
| Dipropylene glycol | 2 |
| Potassium polyoxyethylene alkyl ether acetate | 2 |
| Glycerin | 3 |
| Acrylates / C₁₀₋₃₀ Alkyl Acrylate Crosspolymer ^{a)} | 0.2 |
| Cocamide propyl betaine | 8 |
| Ethylene glycol distearate | 2 |
| Salicylic acid | 0.2 |
| BHT | 0.2 |
| Perfume | 0.05 |
| Purified water | Balance |
| Total | 100.0 |

| | |
|---|---|
| a) "Carbopol ETD2020"; product of BFGoodrich | |

The body shampoos obtained in Examples 15 and 16 were excellent in foamability and foam quality and at the same time, gave a good feel after use.

### Example 17 (Face wash)

A face wash was prepared according to the following formulation.

| | (parts by weight) |
|---|---|
| Potassium lauryl phosphate/potassium dilauryl phosphate (monolauryl phosphate / dilauryl phosphate = 75/25) | 19 |
| Sodium polyoxyethylene lauryl ether sulfate | |
| (t = 2 moles) | 5 |
| Dipropylene glycol | 1.5 |
| Propylene glycol | 5 |
| Sorbitol | 5 |
| Potassium polyoxyethylene alkyl ether acetate (alkyl group: C₁₂/C₁₄ = 75/25, EO:10) | 1.8 |
| Polyoxyethylene sorbitan triisostearate (EO=160) | 2 |
| Cationized cellulose ("Poiz C-150L", product of Kao Corporation) | 0.3 |
| Acrylates / C₁₀₋₃₀ Alkyl Acrylate Crosspolymer ^{a)} | 0.5 |
| Lauryl hydroxy sulfobetaine | 3 |
| Lauramide propyl betaine | 2 |
| Ethylene glycol distearate | 2 |
| Methylparaben | 0.2 |
| Propylparaben | 0.1 |
| BHT | 0.2 |
| Perfume | 0.05 |
| Purified water | Balance |
| Total | 100.0 |

| | |
|---|---|
| a) "Carbopol ETD2020"; product of BFGoodrich | |

### Example 18 (Face wash)

A face wash was prepared in accordance with the following formulation.

| | (parts by weignt) |
|---|---|
| Triethanolamine lauryl phosphate/triethanolamine dilauryl phosphate (monolauryl phosphate / dilauryl phosphate = 75/25) | 19 |
| Triethanolamine polyoxyethylene lauryl ether sulfate (t = 3 moles) | 2.5 |
| Triethanolamine myristate | 2.5 |
| Glycerin | 16 |
| Polyoxyethylene sorbitan triisostearate (EO=160) | 2 |
| Cationized cellulose ("Poiz C-150L", product of Kao Corporation) | 0.5 |
| Acrylates / C₁₀₋₃₀ Alkyl Acrylate Crosspolymer ^{a)} | 0.5 |
| Lauramide propyl betaine | 4 |
| Methylparaben | 0.2 |
| Propylparaben | 0.1 |
| BHT | 0.2 |
| Purified water | Balance |
| Total | 100.0 |

| | |
|---|---|
| a) "Carbopol ETD2020"; product of BFGoodrich | |

The face washes prepared in Examples 17 and 18 were excellent in foamability and foam quality, and at the same time gave a good feel after use.

### Example 19 (Body Shampoo)

The body shampoo was prepared in accordance with the following formulation.

| | (parts by weight) |
|---|---|
| Potassium polyoxyethylene (2) lauryl phosphate/ | |
| potassium polyoxyethylene (2) dilauryl phosphate (monolauryl phosphate / dilauryl phosphate = 75/25) | 15 |
| Sodium polyoxyethylene lauryl ether sulfate (t = 2 moles) | 5 |
| Propylene glycol | 5 |
| Cationized cellulose ("Poiz C-150L", product of Kao Corporation) | 0.3 |
| Lauryl hydroxy sulfobetaine | 5 |
| Ethylene glycol distearate | 2 |
| Methylparaben | 0.2 |
| Propylparaben | 0.1 |
| BHT | 0.2 |
| Perfume | 0.05 |
| Purified water | Balance |
| Total | 100.0 |

The body shampoo obtained in Example 19 was excellent in foamability and foam quality and at the same time, gave a good feel after use.

### Example 20 (Face wash)

A face wash was prepared in accordance with the following formulation.

| | (parts by weight) |
|---|---|
| Potassium polyoxyethylene (2) lauryl phosphate/potassium polyoxyethylene (2) dilauryl phosphate (monolauryl phosphate / dilauryl phosphate = 75/25) | 19 |
| Sodium polyoxyethylene lauryl ether sulfate (t = 2 moles) | 5 |
| Dipropylene glycol | 1.5 |
| Propylene glycol | 5 |
| Sorbitol | 5 |
| Potassium polyoxyethylene alkyl ether acetate (alkyl group: C₁₂/C₁₄ = 75/25, EO: 10) | 1.8 |
| Polyoxyethylene sorbitan triisosrearate (EO=160) | 2 |
| Cationized cellulose ("Poiz C-150L", product of Kao Corporation) | 0.3 |
| Acrylates / C₁₀₋₃₀ Alkyl Acrylate Crosspolymer ^{a)} | 0.5 |
| Lauryl hydroxy sulfobetaine | 3 |
| Lauramide propyl betaine | 2 |
| Ethylene glycol distearate | 2 |
| Methylparaben | 0.2 |
| Propylparaben | 0.1 |
| BHT | 0.2 |
| Perfume | 0.05 |
| Purified water | Balance |
| Total | 100.0 |

| | |
|---|---|
| a) "Carbopol ETD2020"; product of BFGoodrich | |

The body wash obtained in Example 20 was excellent in foamability and foam quality and at the same time, gave a good feel after use.

### Industrial Applicability

The detergent compositions of the present invention have less irritation to the skin or the like, are excellent in foamability, foam quality and low temperature stability, and after washing therewith, give the skin or the like a moist feeling, and permit easy mixing during preparation.

## Claims

1. A detergent composition comprising the following components (A), (B) and (C):
(A) a phosphate surfactant containing, as a mixture,
(a₁) a phosphate monoester represented by the below-described formula (1), and
(a₂) a phosphate diester represented by the below-described formula (2): 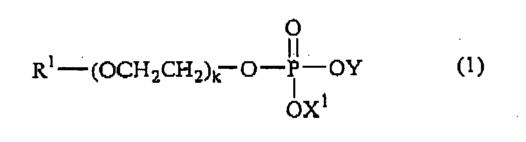 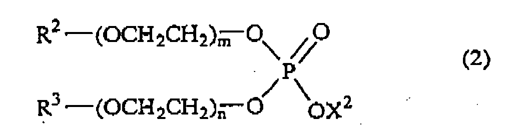 (wherein, R¹, R² and R³ each represents a linear or branched C₈₋₁₈ alkyl or alkenyl group, X¹, X² and Y each represents a hydrogen atom, an alkali metal atom, an alkaline earth metal atom, an alkanolamine or ammonium, and average molar numbers k, m and n each stands for a number of from 0 to 10) at a (a₁)/(a₂) weight ratio of 85/15 to 50/50,
(B) a polyoxyethylene alkyl ether sulfate represented by the following formula (3):
R⁴O(CH₂CH₂O)ₜSO₃M (3)
(wherein, R⁴ represents a saturated or unsaturated C₈₋₁₈ hydrocarbon group, an average molar number t stands for a number of from 0.1 to 5 and M represents an alkali metal atom, alkanolamine or ammonium), and
(C) one or more compounds selected from sulfobetaines represented by the following formula (4): 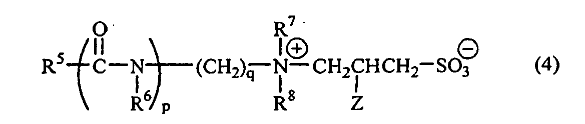 (wherein, R⁵ represents a saturated or unsaturated C₈₋₁₈ hydrocarbon group, R⁶ reprsents a hydrogen atom or a C₁₋₄ alkyl group, R⁷ and R⁸ each represents a C₁₋₄ alkyl group, p stands for a number of from 0 to 1, q stands for a number of from 0 to 6, and Z represents a hydrogen atom or a hydroxyl group) and alkylamide propyl betaines represented by the following formula (5): 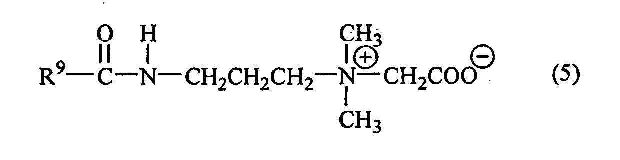 (wherein, R⁹ represents a saturated or unsaturated C₈₋₁₈ hydrocarbon group).

2. A detergent composition of Claim 1, wherein the weight ratio of Component (A) to Component (B),(A)/(B), ranges from 95/5 to 50/5.

3. A detergent composition of Claim 1 or 2, wherein the weight ratio of the sum of Components (A) and (B) to Component (C), [(A) + (B)]/(C), ranges from 99/1 to 40/60.

4. A detergent composition of any one of Claims 1 to 3, wherein the content of Component (A) ranges from 5 to 40 wt.%.

5. A detergent composition of any one of Claims 1 to 4, wherein the content of Component (B) ranges from 1 to 40 wt.%.

6. A detergent composition of any one of Claims 1 to 5, wherein the content of Component (C) ranges from 0.1 to 20 wt.%.

7. A detergent composition of any one of Claims 1 to 3, wherein the contents of Components (A), (B) and (C) range from 5 to 40 wt.%, 1 to 40 wt.% and 0.1 to 20 wt.%, respectively.

8. A detergent composition of any one of Claims 1 to 7, further comprising a cationized polymer as Component (D).

9. A detergent composition of Claim 8, wherein the content of Component (D) ranges from 0.05 to 0.5 wt.%.

10. A process for preparation of a detergent composition as claimed in any one of Claims 1 to 9, which comprises mixing Components (A), (B) and (C), or Components (A), (B), (C) and (D), and stirring the resulting mixture under heating at 40 to 80°C.

## Patentansprüche

1. Reinigungszusammensetzung, umfassend die folgenden Komponenten (A), (B) und (C):
(A) Phosphattensid, umfassend als Mischung:
(a₁) einen Phosphatmonoester, dargestellt durch die unten beschriebene Formel (1), und
(a₂) einen Phosphatdiester, dargestellt durch die unten beschriebene Formel (2): 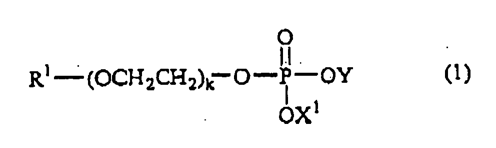 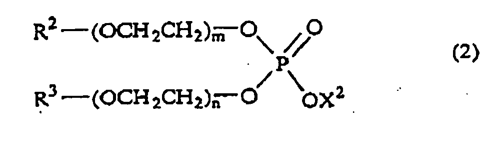
worin R¹, R² und R³ jeweils eine lineare oder verzweigte C₈₋₁₈-Alkyl- oder -Alkenylgruppe sind, X¹, X² und Y jeweils ein Wasserstoffatom, Alkalimetallatom, Erdalkalimetallatom, Alkanolamin oder Ammonium sind und die durchschnittlichen molaren Zahlen k, m und n jeweils für eine Zahl von 0 bis 10 stehen, bei einem Gewichtsverhältnis von (a₁)/(a₂) von 85/15 bis 50/50,
(B) ein Polyoxyethylenalkylethersulfat, dargestellt durch die folgende Formel (3):
**R⁴O(CH₂CH₂O)ₜSO₃M** (3)
worin R⁴ eine gesättigte oder ungesättigte C₈₋₁₈-Kohlenwasserstoffgruppe ist, eine durchschnittliche molare Zahl t für eine Zahl von 0,1 bis 5 steht und M ein Alkalimetallatom, Alkanolamin oder Ammonium ist, und
(C) eine oder mehrere Verbindungen, ausgewählt aus Sulfobetainen mit der folgenden Formel (4): 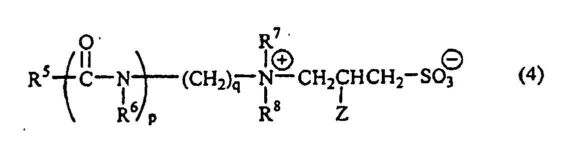
worin R⁵ eine gesättigte oder ungesättigte C₈₋₁₈-Kohlenwasserstoffgruppe ist, R⁶ ein Wasserstoffatom oder eine C₁₋₄-Alkylgruppe ist, R⁷ und R⁸ jeweils eine C₁₋₄-Alkylgruppe sind, p für eine Zahl von 0 bis 1 steht, q für eine Zahl von 0 bis 6 steht und Z ein Wasserstoffatom oder eine Hydroxylgruppe ist, und Alkylamidpropylbetaine mit der folgenden Formel (5) : 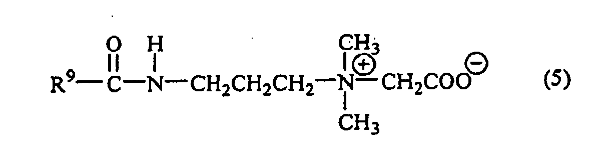 worin R⁹ eine gesättigte oder ungesättigte C₈₋₁₈-Kohlenwasserstoffgruppe ist.

2. Reinigungszusammensetzung nach Anspruch 1, worin das Gewichtsverhältnis der Komponente (A) zur Komponente (B), (A)/(B), im Bereich von 95/5 bis 50/5 liegt.

3. Reinigungszusammensetzung nach Anspruch 1 oder 2, worin das Gewichtsverhältnis der Summe der Komponenten (A) und (B) zur Komponente (C), [(A) + (B)] / (C) im Bereich von 99/1 bis 40/60 liegt.

4. Reinigungszusammensetzung nach einem der Ansprüche 1 bis 3, worin der Gehalt der Komponente (A) im Bereich von 5 bis 40 Gew.% liegt.

5. Reinigungszusammensetzung nach einem der Ansprüche 1 bis 4, worin der Gehalt der Komponente (B) im Bereich von 1 bis 40 Gew.% ist.

6. Reinigungszusammensetzung nach einem der Ansprüche 1 bis worin der Gehalt der Komponente (C) im Bereich von 0,1 bis 20 Gew.% ist.

7. Reinigungszusammensetzung nach einem der Ansprüche 1 bis 3, worin die Gehalte der Komponenten (A), (B) und (C) im Bereich von 5 bis 40 Gew.%, 1 bis 40 Gew.% beziehungsweise 0,1 bis 20 Gew.% liegen.

8. Reinigungszusammensetzung nach einem der Ansprüche 1 bis 7, weiterhin umfassend ein kationisiertes Polymer als Komponente (D).

9. Reinigungszusammensetzung nach Anspruch 8, worin der Gehalt der Komponente (D) im Bereich von 0,05 bis 0,5 Gew.% ist.

10. Verfahren zur Herstellung einer Reinigungszusammensetzung nach einem der Ansprüche 1 bis 9, umfassend das Mischen der Komponenten (A), (B) und (C) oder der Komponenten (A), (B), (C) und (D) und Rühren der resultierenden Mischung unter Erwärmen bei 40 bis 80°C.

## Revendications

1. Composition détergente comprenant les composants suivants (A), (B) et (C) :
(A) un tensioactif de phosphate contenant, en tant que mélange,
(a₁) un monoester de phosphate représenté par la formule (1) décrite ci-dessous, et
(a₂) un diester de phosphate représenté par la formule (2) décrite ci-dessous ; 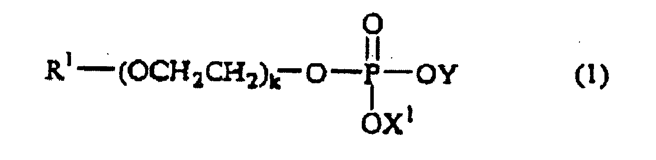 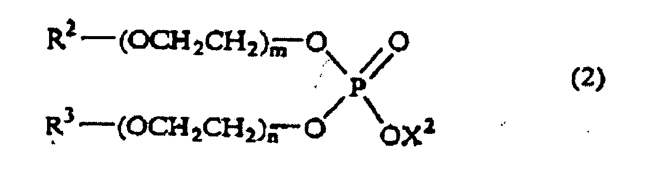 (dans lesquelles R¹, R² et R³ représentent chacun un groupe alkyle ou alcényle en C₈₋₁₈ linéaire ou ramifié, X¹, X² et Y représentent chacun un atome d'hydrogène, un atome de métal alcalin, un atome de métal alcalino-terreux, une alcanolamine ou un ammonium, et les nombres molaires moyens k, m et n correspondent chacun à un nombre de 0 à 10) à un rapport en poids (a₁)/(a₂) de 85/15 à 50/50,
(B) un polyoxyéthylène alkyl éther sulfate représenté par a formule (3) suivante :
R⁴O(CH₂CH₂O)ₜSO₃M (3)
(dans laquelle R⁴ représente un groupe hydrocarbure en C₈-₁₈ saturé ou insaturé, un nombre molaire moyen t correspond à un nombre de 0,1 à 5 et M représente un atome de métal alcalin, une alcanolamine ou un ammonium), et
(C) un ou plusieurs composés choisis parmi les sulfobétaïnes représentées par la formule (4) suivante : 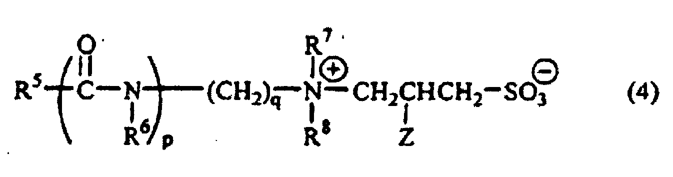 (dans laquelle R⁵ représente un groupe hydrocarbure en C₈₋₁₈ saturé ou insaturé , R⁶ représente un atome d'hydrogène ou un groupe alkyle en C₁₋₄, R⁷ et R⁸ représentent chacun un groupe alkyle en C₁₋₄, p correspond à un nombre de 0 à 1, q correspond à un nombre de 0 à 6, et Z représente un atome d'hydrogène ou un groupe hydroxyle) et les alkylamide propyl bétaïnes représentées par la formule (5) suivante : 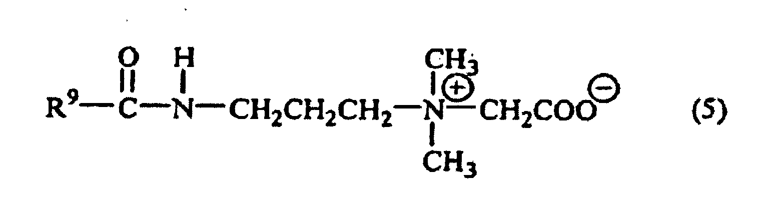 (dans laquelle R⁹ représente un groupe hydrocarbure en C₈₋₁₈ saturé ou insaturé).

2. Composition détergente selon la revendication 1, dans laquelle le rapport en poids du composant (A) au composant (B), (A)/(B), se situe dans la plage allant de 95/5 à 50/5.

3. Composition détergente selon la revendication 1 ou 2, dans laquelle le rapport en poids de la somme des composants (A) et (B) au composant (C), [(A) + (B)] / (C) se situe dans la plage allant de 99/1 à 40/60.

4. Composition détergente selon l'une quelconque des revendications 1 à 3, dans laquelle la teneur en composant (A) se situe dans la plage allant de 5 à 40 % en poids.

5. Composition détergente selon l'une quelconque des revendications 1 à 4, dans laquelle la teneur en composant (B) se situe dans la plage allant de 1 à 40 % en poids.

6. Composition détergente selon l'une quelconque des revendications 1 à 5, dans laquelle la teneur en composant (C) se situe dans la plage allant de 0,1 à 20 % en poids.

7. Composition détergente selon l'une quelconque des revendications 1 à 3, dans laquelle les teneurs en composants (A), (B) et (C) se situent dans les plages allant de 5 à 40 % en poids, de 1 à 40 % en poids et de 0,1 à 20 % en poids, respectivement.

8. Composition détergente selon l'une quelconque des revendications 1 à 7, comprenant en outre un polymère cationisé comme composant (D).

9. Composition détergente selon la revendication 8, dans laquelle la teneur en composant (D) se situe dans la plage allant de 0,05 à 0,5 % en poids.

10. Procédé de préparation d'une composition détergente selon l'une quelconque des revendications 1 à 9, qui comprend le mélange des composants (A), (B) et (C), ou des composants (A), (B), (C) et (D), et l'agitation du mélange résultant sous chauffage à 40 à 80°C.
